# EUROPEAN PATENT APPLICATION

(11) **EP 4 616 941 A1**
(43) Date of publication of application: **17.09.2025**
(21) Application number: 24163917.8
(22) Date of filing: 15.03.2024
(51) Int. Cl.: B01J 20/26

(54) **METHOD FOR REMOVING PER- AND POLYFLUOROALKYL SUBSTANCES**

(71) Applicant: AIRplus GmbH, 83026 Rosenheim (DE)
(72) Inventor: Freilinger, Johanna, Innsbruck (AT); Bakry, Rania, Innsbruck (AT); Schottenberger, Herwig, Innsbruck (AT); Obholzer, Thomas, Innsbruck (AT); Rupprich, Marco, Innsbruck (AT); Färber, Robert, Rosenheim (DE)
(74) Representative: Schwarz & Partner Patentanwälte GmbH

(57) **Abstract**

A polymer of formula I 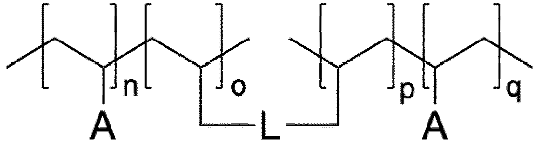 and uses of the polymer for the removal of removing per- and polyfluoroalkyl substances from aqueous solutions.

## Description

The present invention relates to a polymer for removing per- and polyfluoroalkyl substances and to a device including the polymer for removing per- and polyfluoroalkyl substances (PFAS) from aqueous solutions. The invention also relates to devices such as a chromatography column, in particular an HPLC chromatography column, a solid phase extraction (SPE) cartridge, a hemodialysis device or a hemoperfusion device comprising a polymer. Finally, the invention relates to a method for removing per- and polyfluoroalkyl substances (PFAS) from aqueous solution such as tap water or bodily fluids e.g. blood or serum.

### BACKGROUND OF THE INVENTION

Per- and polyfluoroalkyl substances (PFAS) are a group of anthropogenic organic compounds having multiple fluorine atoms attached to an alkyl chain. The group of PFAS includes polymeric and non-polymeric substances, the latter comprising fluorinated surfactants. The widespread use of fluorinated surfactants and fluorinated acids such as perfluorooctane sulfonic acid (PFOS) and perfluorooctanoic acid (PFOA) has resulted in their release into the environment and the accumulation in water including ground and drinking water.

PFAS are persistent chemicals that accumulate in organisms, in particular in humans and animals and so the environmental sequestration of these PFAS from aqueous media by adsorption is a pressing and challenging problem which necessitates materials science to offer sustainable solutions. As of today, PFAS are mostly sorbed by activated carbon, which is a suitable, but not very selective adsorbent for PFAS. One drawback of activated carbon is the fact that the sorption is irreversible and so new cartridges with activated carbon must be installed once the activated carbon is saturated.

In contrast to widely utilized carbonaceous sorption materials (Zhi, Y.; Liu, J. Adsorption of perfluoroalkyl acids by carbonaceous adsorbents: Effect of carbon surface chemistry. Environ. Pollut. 2015, 202, 168-176, doi:10.1016/j.envpol.2015.03.019), ion exchange (IEX) is a more flexible and promising technology to remove legacy ionic PFAS from water (Liu, Y.-L.; Sun, M. Ion exchange removal and resin regeneration to treat per- and polyfluoroalkyl ether acids and other emerging PFAS in drinking water. Water Res. 2021, 207, 117781, doi:10.1016/j.watres.2021.117781).

In terms of tailoring the sorption efficiency and selectivity of sequestrants for PFAS, IEX belongs to the most advantageous methodologies for their sequestration from aquatic sources (de Dardel F, Arden TV. Ion exchangers. In: Ullmann's Encyclopedia of Industrial Chemistry. Weinheim, Germany: Wiley-VCH Verlag GmbH & Co. KGaA; 2000. doi:10.1002/14356007.a14_393.pub2).

Expectedly, anion-exchange resins exhibit better adsorption of PFAS compared to cation-exchange resins, nonionic resins, and granulated active carbon, regardless of the PFAS's predicted charge (Fang, Y.; Ellis, A.; Choi, Y.J.; Boyer, T.H.; Higgins, C.P.; Schaefer, C.E.; Strathmann, T.J. Removal of Per- and Polyfluoroalkyl Substances (PFASs) in Aqueous Film-Forming Foam (AFFF) Using Ion-Exchange and Nonionic Resins. Environ. Sci. Technol. 2021, 55, 5001-5011, doi:10.1021/acs.est.1c00769). A strong relationship between the hydrophobicity of the exchange functional group of the resin and its capacity in removing PFAS exists.

EP 3 442 911 A1 discloses a method and system for removing and concentrating per- and polyfluoroalkyl substances (PFAS) from water by means of an anion exchange resin.

Manning et al. (Irene M. Manning, Nick Guan Pin Chew, Haley P. Macdonald, Kelsey E. Miller, Mark J. Strynar, Orlando Coronell, and FrankA. Leibfarth; Hydrolytically Stable Ionic Fluorogels for High-Performance Remediation of Per- and Polyfluoroalkyl Substances (PFAS) from Natural Water; Angew. Chem. Int. Ed. 2022, 61, https://onlinelibrary.wiley.com/doi/10.1002/anie.202208150) describe PFAS sorption based on ionic fluorogels with fluorinated phenoxy groups.

Singh et al. (Anuradha Singh, Richard Lynch, Jacob Solomon, Jimmie D. Weaver, Alexa R. May, Development of novel fluor mop materials for remediation of perfluoroalkyl substances (PFAS) from groundwater, J. Hazard. Mater., 2023, 448, 130853; https://doi.org/10.1016/j.jhazmat.2023.130853) show removal of different PFAS with functionalized silica materials. The functionalized silica materials include polyfluorinated alkyl chains attached to SiO₂ via an imidazolium group such as N-(3,3,4,4,5,5,6,6,7,7,8,8,9,9,10,10,10-Heptadecafluorodecyl) imidazole (FMV8). The functionalized silica materials show a selective and effective removal of PFAS from impacted groundwater. However, the materials disclosed by Singh et al. suffer from the weak and hydrolytically sensitive covalent bonds of polyfluoroalkyl chained imidazolium linkers. As a consequence, these materials may also release PFAS due to leaching of hydrolytic degradation products into the materials targeted for PFAS sequestration.

Chavan et al. (Santosh N. Chavan, Anil K. Padhan and Debaprasad Mandal; Self-assembly of fluorous amphiphilic copolymers with ionogels and surface switchable wettability; Polym. Chem., 2018, 9, 2258-2270) disclose fluorous amphiphilic copolymers with repeating imidazole units and perfluorinated alkyl residues.

Principal restrictions such as low selectivity, or low sensitivity towards functional groups, permanent ionic character, and general limited reusability are among the most important existing disadvantages in the prior art. In addition, regenerants release only short-chain PFAS acids in classic IEX resins unless organic solvents are used. For example, the exploitation of hydrophobic cyclodextrin pockets, combined with fluorophilic moieties or calixarenes are working sufficiently, however, they require particularly high and cost-intensive synthetic efforts, and thus, unfortunately, disqualify themselves for a broad practical application. Other methods and devices such as proposed by Singh et al. also have drawbacks with regard to the attachment of the sorbing functionalities to a carrier backbone. At present, these limitations cannot be overcome even by disintegrative physical methods of PFAS removal.

### BRIEF DESCRIPTION OF THE INVENTION

All remediation methods for PFAS and all compounds and devices used for this purpose according to the prior art suffer from drawbacks which limit their practical implementation. Known methods and devices show insufficient selectivity and limited reusability or may even release PFAS on their own if used improperly.

Having regard to the state of the art, the technical problem underlying the present invention consisted in developing a compound, device, and method for selectively and reversibly sorbing PFAS without the drawbacks of the prior art.

The technical problem outlined above is solved by a polymer according to formula I 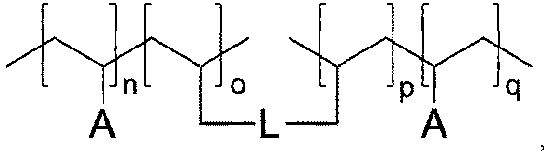 wherein
n, o, p, and q are indenpendently integers ≥ 1,
wherein
A= 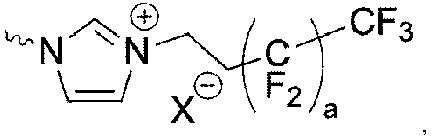
wherein X = Cl, Br, I and *a* is an integer from 1 to 8,
L= 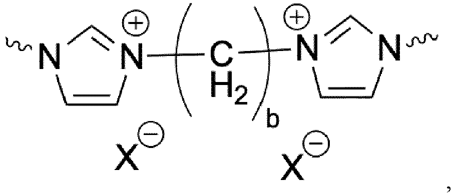
wherein X = Cl, Br, I and b is 1, 3, 4, 5, or 6,
or L = 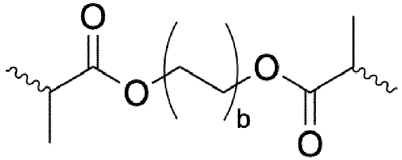
wherein b is 1, 2, or 4.

In preferred embodiments the ratio of n : o = 1 : (0,8 to 1,2) and the ratio of p : q = 1 : (0,8 to 1,2).

In another preferred embodiment a = 3 or 5, in particular 5.

In another preferred embodiment, if L= 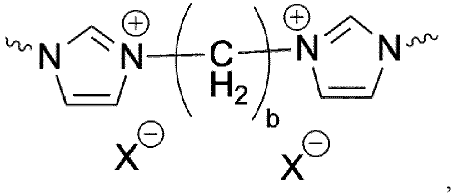 then b =1, 3, 4, 5, 6.

Preferably, in all variants comprising X⁻, X = Cl or Br.

In a preferred embodiment, the polymer of formula I has the following structure: 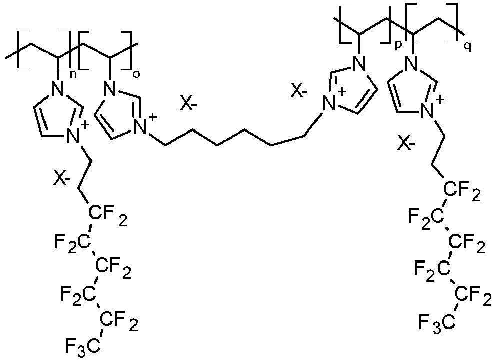 with n, o, p, and q are indenpendently integers ≥ 1, and X = Cl, Br, I.

The technical problem is also solved by a method for removing per- and polyfluoroalkyl substances (PFAS) from aqueous solution, the method comprising contacting the aqueous solution containing PFAS with a polymer as defined in formula I, the polymer thereby adsorbing the PFAS.

The PFAS to be removed is water soluble and an acid (i.e. it has an acid functionality) as defined below.

Furthermore, the technical problem outlined above is solved by a device for removing per- and polyfluoroalkyl substances (PFAS) from aqueous solution, the device comprising a housing with an inlet and an outlet for water, wherein the housing comprises a polymer of formula I.

In one embodiment the device is a chromatography column, in particular an HPLC chromatography column, comprising a stationary phase, wherein the stationary phase comprises a polymer as defined above, preferably wherein the stationary phase is a polymer of formula I.

In one embodiment, the invention relates to a solid phase extraction (SPE) cartridge, comprising a stationary phase with a polymer as defined in formula I.

In other embodiments, the device can be a hemodialysis device or a hemoperfusion device for removing per- and polyfluoroalkyl substances (PFAS) from an extracorporeal bodily fluid such as blood sample or serum sample, the device comprising a fluid circuit comprising an inlet and an outlet;
a blood pump for circulating the fluid between the inlet and the outlet;
a purification device arranged in the fluid circuit between the inlet and the outlet;
wherein the blood purification device comprised a polymer of formula I.

By this, the ion exchangers can also be used in the form of PFAS specific adsorbents entrapped inside artificial cell membranes.

Hemoperfusion is an extracorporeal procedure for the elimination of toxins that is used in acute poisoning. It is indicated if the toxin cannot be adequately eliminated from the body by hemodialysis or hemofiltration (see e,g, Ronco, C., Bellomo, R. Hemoperfusion: technical aspects and state of the art. Crit Care 26, 135 (2022). https://doi.org/10.1186/s13054-022-04009-w).

Hemodialysis is a procedure where a hemodialysis device comprises a purification device to clean blood or plasma (see e.g. Fazal-Ur-Rehman, Khan, S.N. (2012). Application of Ion Exchange Resins in Kidney Dialysis. In: Inamuddin, D., Luqman, M. (eds) Ion Exchange Technology II. Springer, Dordrecht. https://doi.org/10.1007/978-94-007-4026-6_8).

All of the mentioned aspects of the invention have in common that they involve the polymer of formula I. This polymer is capable of selectively and reversibly sorbing PFAS because it has two functionalities that synergistically promote specific adsorption of different PFAS, in particular water soluble PFAS acids. Due to the two different cooperative functional moieties, the adsorption is reversible in the sense that a once bound PFAS compound can be rendered easier removable by turning off one of the dual mode affinity mechanisms.

In particular, the sorption of PFAS on the polymers is based on two synergistic mechanisms: Positive imidazolium ionic charges that allow for an ionic (electrostatic) bonding in the sense of IEX on the one hand, and the interaction by azolium-attached fluorinated alkyl chains, thus providing a hydrophobic, in particular fluorophilic association on the other hand. Furthermore, both mechanisms occur at a single site on the polymer, establishing a synergistic dual bonding mechanism in contrast to otherwise - albeit cooperative - but only remote sorption sites.

The polymer contains repetitive units bearing a cationic group with covalently attached fluorinated alkyl chains. The fluorophilic behavior should not be confused with hydrophobicity in general, since fluorosurfactants are liking water only at their polar head, but the hydrophobic tail not only dislikes polar (aqueous) environments, but also, to some lesser extent, exhibits only low affinity to ordinary organic scaffolds, including polyacrylic or polystyrenic aromatic hydrocarbons of common IEX-resins as well. This means that the apolar fluoroponytail is omniphobic that is repulsive to all other types of (non-fluorous) chemical constituents. While Singh et al. (cited above) shows a binding mechanism with fluorinated alkyl chains, the most important difference is that the present invention relates to polymers whereas Singh et al. use hydrolytically sensitive, PFAS-leachable, silica grafted monomeric units comprising the fluorinated alkyl chain. The present invention not only results in a more stable sorbent material, it also allows a polymer to be tailored in terms of specific sorption situations, and consequently, due to the close proximity of the two cooperative functional groups, the binding is stronger and the selectivity is higher.

In summary, both, the fluorine - fluorine interactions between fluorous side chain segments of the polymer as a sorbent and the fluorinated tail of a PFAS (sequestrant), as well as electrostatic attraction between quaternized (or protonated) ammonium or azolium groups (sorbent), and the anionic fluorosurfactant headgroups are crucial to achieve effective PFAS sorption from aqueous solutions with pronounced PFAS selectivity. The fluorine - fluorine interactions contribute to recognition of PFAS molecules via fluorophilicity, with fast exchange observable between bound and free PFAS, while the electrostatic interactions can tightly bind acidic/anionic fluorosurfactants, thus precluding such accelerated exchange equilibria. It was shown that the resinous sorbents according to formula I containing both fluorinated and cationic aromatic groups have a higher PFAS removal efficiency with potentially improved sorption capacity compared with the sorbents with a single functional group, and that the electrostatic attraction is stronger and dominates the fluorine - fluorine interactions when the sorbent is highly charged.

Depending on the pH value of the solvent surrounding the polymer of formula I, acid base equilibriua can be operative between the quarternized imidazolium group (protocarbenes; A) and imidazole-2-ylidenes (a subgroup of nitrogen heterocyclic carbenes, NHCs; B). Therefore, the latter are easily obtained by deprotonation of the C2-H, using bases. E.g., triethylamine is able to promote C2-H exchange. Even acetate as a moderately basic counter-ion is able to spontaneously form NHCs:

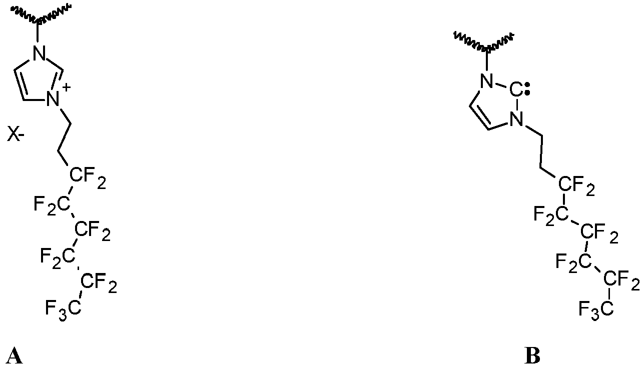

To allay fears that NHC hydrolysis products could be cleaved from the polyvinylimidazolium-based sorbent polymers of formula I, which in turn could also spill unwanted PFAS into the environment, it has to be noted that the structure of the polymer ensures that any ring opening hydrolysis products stemming from preformed NHC or corresponding NHC-adducts (such as CO₂, forming zwitterionic carboxylates) would retain the fluoroponytail at the polymer backbone. Further, a dynamic back-reaction equilibrium in aqueous environment takes place to reconstitute the starting imidazolium hydroxide anyway.

One advantage of the present invention is the straightforward desorbability of the PFAS and thus the easy regeneration of the polymer. An alcohol and/or water solution of a base or corresponding basic salt is sufficient. E.g. an alcohol and/or water solution of NaCl can be used. The solution is preferably acidic (pH preferably 8 to 12) or basic (pH preferably 2 to 6).

The polymer can be obtained by polymerizing / crosslinking a vinyl compound with a divinyl compound.

The vinyl compound can be: 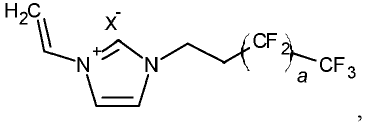 wherein a is an integer from 1 to 6 and X⁻ = Cl⁻, Br⁻, I⁻.

Preferably, the vinyl compound is selected from the group: 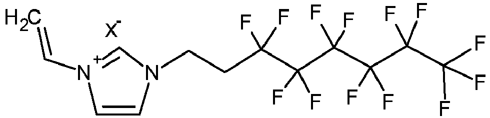 or 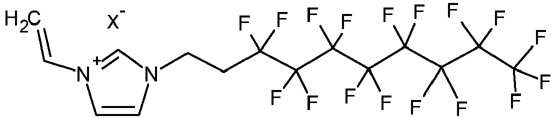 wherein in both examples X⁻ = Cl⁻, Br⁻, I⁻

The divinyl compound can be 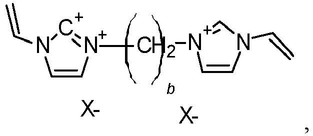 wherein b = 1, 3, 4, 5, or 6 and X⁻ = Cl⁻, Br⁻, I⁻

Preferably, the divinyl compound is selected from the group: 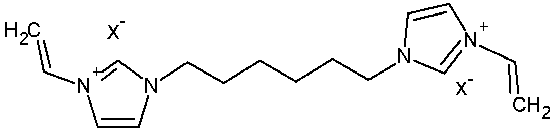 wherein X⁻ = Cl⁻, Br⁻, I⁻
or

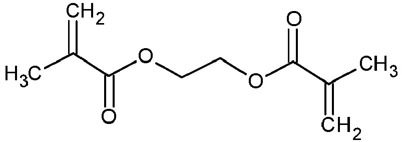

The molar ratio between the vinyl compound and the divinyl compound before crosslinking is preferably in the range of 1 : 0,8 to 1,2.

### Definitions:

**PFAS:** PFAS is an abbreviation for per- and polyfluorinated chemicals. PFAS do not occur naturally and have only been produced and used since the late 1940s. PFAS relevant for the invention consist of carbon chains, typically alkyl groups, of various lengths in which the hydrogen atoms are completely (perfluorinated) or partially (polyfluorinated) replaced by fluorine atoms. Perfluorinated carbonic and sulphonic acids and their precursor compounds are used most frequently and are the PFAS most prominently aimed at by the invention. The most important PFAS in the sense of the invention are PFOS, PFOA, and hexafluoropropylene oxide dimer acid (HFPO-DA) and the ones listed in the following table 1. The invention is useful in the removal of these PFAS.

**Table 1: Most common PFAS released into nature.**

| **Name** | **CAS-No** | **Abbreviation** |
|---|---|---|
| Perfluorobutanoic acid | 375-22-4 | PFBA, PF4C |
| Perfluoropentanoic acid | 2706-90-3 | PFPeA, PF5C |
| Perfluorohexanoic acid | 307-24-4 | PFHxA, PF6C |
| Perfluoroheptanoic acide | 375-85-9 | PFHpA, PF7C |
| Perfluorooctanoic acid | 335-67-1 | PFOA, PF8C |
| Perfluorononic acid | 375-95-1 | PFNA, PF9C |
| Perfluorobutane sulfonic acid | 375-73-5 | PFBS, PF4S |
| Perfluoropentanesulfonic acid | 630402-22-1 | PFPeS ,PF5S |
| Perfluorohexanesulfonic acid | 355-46-4 | PFHxS, PF6S |
| Perfluoroheptane sulfonic acid | 375-92-8 | PFHpS, PF7S |
| Perfluorooctane sulfonic acid | 1763-23-1 | PFOS, PF8S |
| Perfluoronanesulfonic acid | 98789-57-2 | PFNS, PF9S |
| Perfluorodecanesulfonic acid | 335-77-3 | PFDS, PF10S |
| Perfluoroundecanesulfonic acid | 749786-16-1 | PFUnDS, PF11S |
| Perfluorododecanesulfonic acid | 79780-39-5 | PFDoDS, PF12S |
| Perfluorotridecanesulfonic acid | n.a. | PFTriDS, PF13S |
| Perfluorooctanesulfonic acid amide | 754-91-6 | PFOSA |
| 6:2 Fluorotelomer sulfonic acid | 27619-97-2 | 6:2 FTS/H4PFOS |
| 8:2 Fluorotelomer sulfonic acid | 39108-34-4 | 8:2 FTS/H4-PFDeS |
| 4:2 Fluorotelomer sulfonic acid | 757124-72-4 | 4:2 FTS |
| perfluoro-4,8-dioxa-3H-nonanoic acid | 919005-14-4 | DONA |
| Perfluoro-2-propoxypropanoic acid | 13252-13-6 | GenX |
| 9-chlorohexadecafluoro-3-oxanonane-1- sulfonic acid | 73606-19-6 | F-53B |

| | | |
|---|---|---|
| See e.g. https:/ /www.umweltbundesamt.at/angebot/analytik/substanzen-im -fokus/poly-perfluorierte-substanz (as of March 13, 2024) | | |

**Water soluble:** Water soluble according to the invention means a solubility of ≥ 0.1 mol/L at 20 °C.

**Acid functionality:** An acid functionality means that the PFAS is capable of donating a proton (i.e. hydrogen ion, H⁺), known as a Bronsted-Lowry acid, in particular it means a carboxylic acid. According to the invention when referring to PFAS, the acid functionality is particularly a sulphonic acid or carbonic acid.

### DETAILED DESCRIPTION OF THE INVENTION

Further details and advantages of the invention are provided along with examples and the attached figures and description of the figures.
- Fig.1a, 1b: schematically shows the PFOS sorption at the polymer.
- Fig.2: shows an amine-anchorable / graftable fluorous zwitterion.
- Fig. 3: shows the ion capacity of polymers with different monomer/crosslinker ratios according to the invention.
- Fig. 4: shows a chromatogram of PFAS before sorption, after sorption and after elution from the polymer.
- Fig. 5: shows adsorption properties of PFAS acids onto a polymer according to the invention.

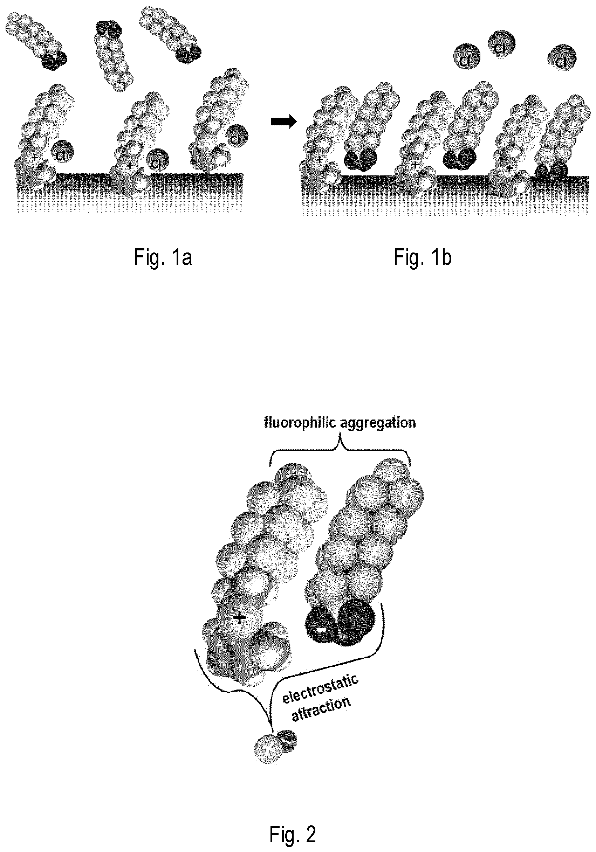

A representative polymer with the functional group is shown in the lower section of Fig. 1a and 1b. PFOS and perfluorobutyric acid with their perfluoroalkyl(ether; Gen X) tails are interacting as ion pairs with the fluorinated alkyl residues of repetitive units of a perfluorohexylethyl-poly-vinylimidazolium resin covalently bound to the polymer through supportive binding in the form of simultaneous fluorophilicity resulting in a disproportionately higher backbone affinity. A maximized cooperative sorption mechanism due to the close proximity of the synergistic functionality of imidazolium and the fluorinated alkyl residues is achieved. Hence, the combined fluorine - fluorine interactions between fluorous segments of the polymer and the fluorinated tail of fluorosurfactants, as well as electrostatic attraction between the ammonium group and the acid residue results in maximum (cumulated / synergistic) interaction.

### 1. Experimental Section:

### 1.1 Polymer Synthesis

40 mg 2,2-dimethoxy-2-phenylacetophenone, 400 mg 3-(perfluorohexyl-ethyl)-1-vinylimidazolium chloride (FP-VIM) [2126844-17-3], 400 mg 3-hexane-1-6-diyl-bis-1-vinyl-1H-imidazol-3-ium dibromide, 500 mg 1-propanol, 500 mg acetonitrile (ACN) and 200 mg H₂O were sonicated for 5 min, inserted between two glass plates and polymerized with UV light for 30 min. 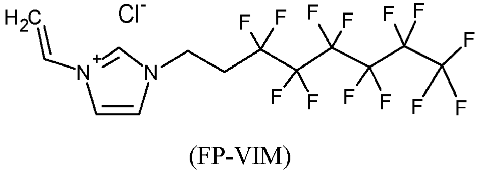 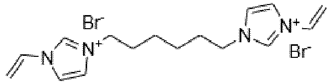 3-hexane-1-6-diyl-bis-1-vinyl-1H-imidazol-3-ium dibromide

The result is a polymer of formula I with A= 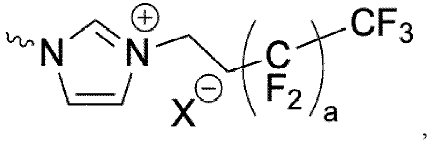
wherein X = Cl and *a* is 5, and L= 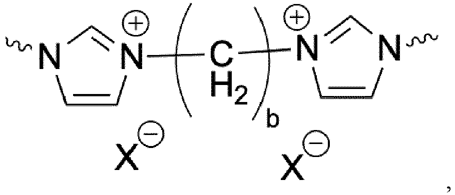
wherein X = Br and b is 6,
wherein the ratio of n : o and p : q is around 1 each.

Due to the high fluorophilicity, the polymer is insoluble in common solvents. As a consequence, the determination of the molecular weight or degree of polymerization and crosslinking is very difficult and can only be guessed. However, the experiments show that the effect of sorption of PFAS is independent of the molecular weight and the degree of polymerization and crosslinking.

### 1.2 Resin preparation for SPE validation

15 mg polymer of formula I according to chapter 1.1 were placed into a 2 mL Eppendorf vial. 1 mL MeOH was added, and the vial incubated in a thermomixer at RT and 1400 rpm for 5 min. Afterwards it was centrifuged at 14 000 rpm and the supernatant removed. The polymer was equilibrated with 1 mL water for 5min at RT and 1400 rpm and then centrifuged for 5 min. Once the supernatant was removed, 1 mL of sample was loaded and incubated for 5 minutes.

### 1.3 Loading protocol SPE

15 mg of crosslinked polymer as obtained above were placed between two polyethylene frits (20 µm porosity) in a 1 cm³ cartridge (Sigma-Aldrich, order number: 54220-U) and pressed with a weight of 12-13 kg using a force meter with a fitting stamp. The solvents were passed through the cartridge using pressurized air at 1 bar with a drop speed of 1 drop per 2 seconds. For conditioning 1 mL of MeOH and 1 mL of were used. Afterwards, 1 mL of solution containing 10 µg/l of PFBA, HFPO-DA (GenX), PFOA, and PFOS was loaded onto the cartridge with a speed of 1 drop / 2 sec. The material was washed with 1 mL of H₂O and elution was achieved with 1 mL of alkaline or ammonium hydroxide methanolic solution. The supernatant was removed with a gentle N₂ stream and the PFAS resuspended in 1 mL containing 10µg·L^{-1 13}PFOA and ¹³PFOS internal standard. Analysis was performed on a Waters Acquity Premier liquid chromatograph coupled to a Waters Xevo TQD triple quadrupole.

### 1.4 Ion Capacity of the Polymer

The ion capacity of polymer of formula I with a variation of the ratio between FP-VIM monomer [2126844-17-3], and crosslinker [1046127-79-0] was determined by placing 25 mg of polymer (particle size between 50-500 µm) between two polyethylene frits (20 µm porosity) in a 1 cm³ cartridge (Sigma-Aldrich, order number: 54220-U). It was compacted with a weight of 12-13 kg using a force meter with a fitting stamp. The polymer was first washed with 3 mL of a 0.1 M NaCl solution followed by 5 mL of H2O. Afterwards, 3 mL of a 0.1 M NaI solution were added to substitute the original halide ions (chloride / bromide stemming from the polymerized monomer salts) of the ion exchange resin. The dripping speed of every solution was 1 drop / 2sec. Measurements were conducted with a Dionex IC-2500 system with AS40 automated sampler, LC25 chromatography oven, GS50 gradient pump and a CD25 conductivity detector.

### 1.5 PFAS Loading Capacity of the Polymer

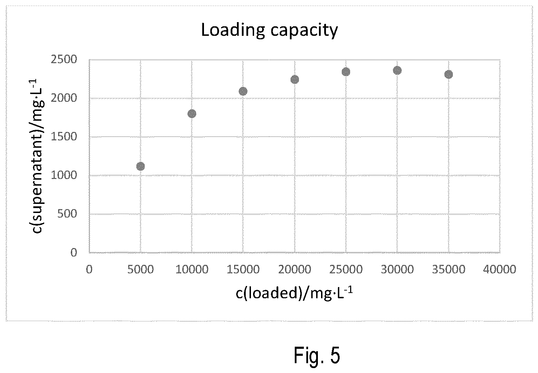

10 mg of the polymer were placed between two polyethylene frits in a cartridge and packing with a force meter with a weight of 12-13 kg. Conditioning was achieved with 1 mL of MeOH and 1 mL water. Afterwards 7 mL of a 5000 mg·L⁻¹ PFOA solution were loaded onto the cartridge and every milliliter was collected in a separate vial. The solvents were passed through the cartridge using pressurized air at 1 bar with a drop speed of 1 drop per 2 seconds. The concentration found in the supernatant is depicted in Fig. 5. Furthermore, the concentration of the supernatant, the adsorbed concentration, as well as the adsorbed mass is listed in Table 3.

### 2. Results:

### 2.1. Ion capacity of the polymer

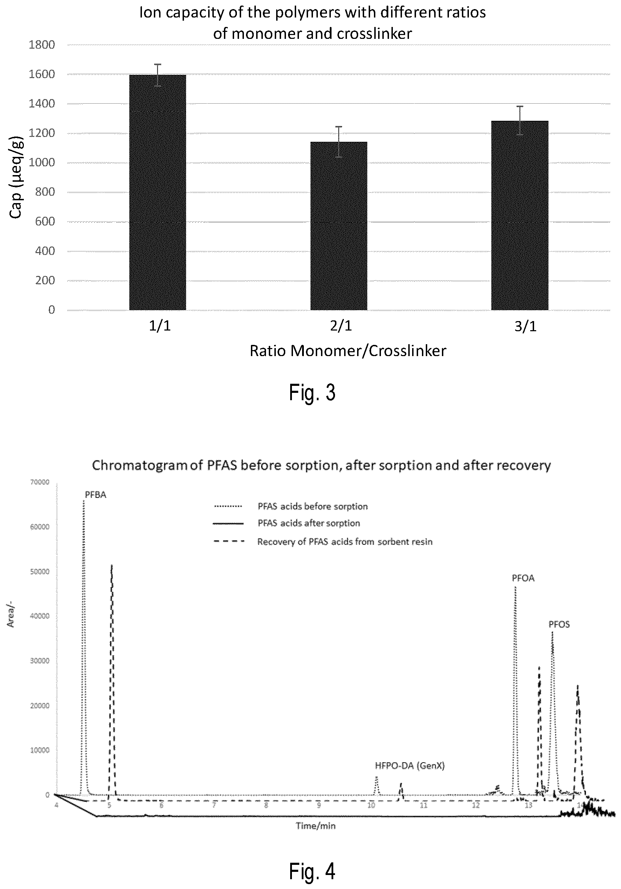

The results of the determined ion capacity are shown in table 2 and depicted in Fig. 3.

**Table 2: Mean and standard deviation values of the polymers with different monomer/crosslinker ratios**

| Ratio Monomer (vinyl compound) /Crosslinker (divinyl compound) | Cap Cl⁻ (µeq/g) | Cap Br⁻ (µeq/g) | Σ Cap (µeq/g) |
|---|---|---|---|
| 1/1 | 943.8± 153 | 649.7± 193 | 1594± 75 |
| 2/1 | 679.2± 62 | 462.1± 43 | 1141± 103 |
| 3/1 | 1003± 87 | 282.5± 17 | 1286± 96 |

The chromatograms of PFAS before sorption, after sorption, as well as after recovery from the polymer is depicted in Fig. 4. It can be shown that PFAS were fully adsorbed onto the polymer. Furthermore, the chromatogram of the recovery shows that 90% PFBA, 88% HFPO-DA, 95% PFOA, and 90% PFOS can be recovered which proves that regeneration of the material can be achieved.

### Loading capacity of the polymer of formula I

**Table 3: Summary of the concentrations in the supernatant and adsorbed PFOA onto the material in each loading step.**

| c (loaded)/ mg·L⁻¹ | c (supernatant)/ mg·L⁻¹ | c (adsorbed)/ mg·L⁻¹ | m (adsorbed)/mg |
|---|---|---|---|
| 5000 | 1117 | 3883 | 3.88 |
| 10000 | 1798 | 3202 | 3.20 |
| 15000 | 2089 | 2911 | 2.91 |
| 20000 | 2241 | 2759 | 2.76 |
| 25000 | 2341 | 2659 | 2.66 |
| 30000 | 2359 | 2641 | 2.64 |
| 35000 | 2308 | 2692 | 2.69 |
| Σ | **14251** | **20749** | **20.7** |

## Claims

1. A polymer of formula I 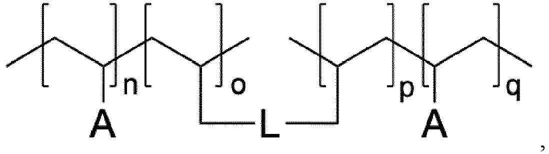 wherein
n, o, p, q are indenpendently integers ≥ 1,
wherein
A= 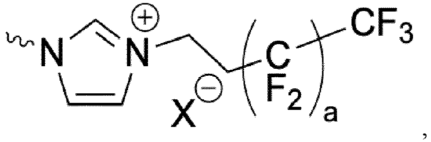
wherein X = Cl, Br, I and *a* is an integer from 1 to 8,
L= 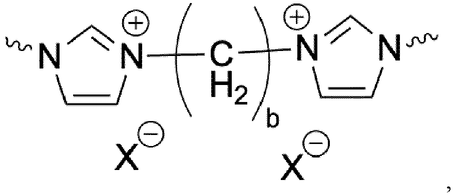
wherein X = Cl, Br, I and *b* is 1, 3, 4, 5, or 6
or L = 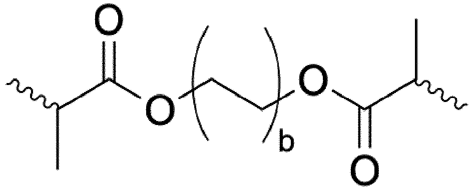
wherein *b* is 1, 2, or 4.

2. A polymer according to claim 1, wherein the ratio of n : o = 1 : (0,8 to 1,2) and p : q = 1 : (0,8 to 1,2) and / or wherein a = 3 or 5, in particular 5 and / or wherein *b* = 6 if L=

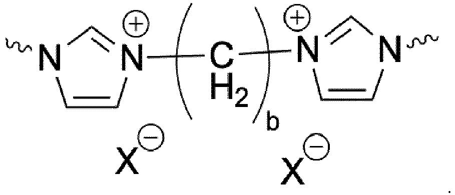

3. A polymer according to formula:

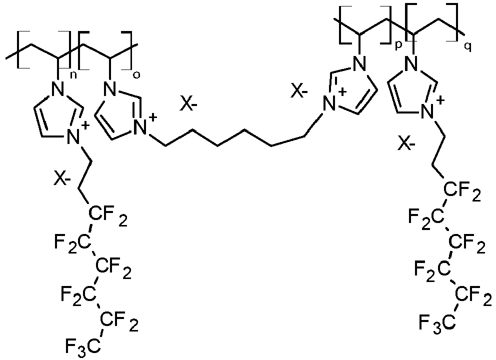

4. A device for removing per- and polyfluoroalkyl substances (PFAS) from water, the device comprising a housing with an inlet and an outlet for water, wherein the housing comprises a polymer according to one of claims 1 to 3.

5. A device according to claim 4, wherein the polymer is kept inside the housing by a filter adjacent to the inlet and a filter adjacent to the outlet.

6. A device according to claim 5, wherein the filter is a frit, preferably a ceramic or glass frit, a metal mesh, preferably a titanium or steel mesh or a membrane of Polytetrafluorethylene.

7. A device according to one of claims 4 to 6, wherein the device is integrated in a water filter cartridge.

8. A chromatography column, in particular an HPLC chromatography column, or a solid phase extraction cartridge, comprising a stationary phase, wherein the stationary phase is a polymer according to one of claims 1 to 3.

9. A hemodialysis device or a hemoperfusion device for removing per- and polyfluoroalkyl substances (PFAS) from an extracorporeal bodily fluid such as blood sample or serum sample, the device comprising a fluid circuit comprising an inlet and an outlet;
a blood pump for circulating the fluid between the inlet and the outlet;
a purification device arranged in the fluid circuit between the inlet and the outlet;
wherein the blood purification device comprised a polymer according to one of claims 1 to 3.

10. A method for removing per- and polyfluoroalkyl substances (PFAS) from an aqueous solution, the method comprising contacting the aqueous solution containing PFAS with a polymer according to one of claims 1 to 3 and thereby adsorbing the PFAS to the polymer.

11. A method according to claim 10, wherein the aqueous solution is an extracorporeal bodily fluid, preferably serum or blood or blood derivative.

12. A method according to claim 10 or claim 11, wherein the PFAS is selected from the group consisting of
Perfluorobutanoic acid; Perfluoropentanoic acid; Perfluorohexanoic acid; Perfluoroheptanoic acide; Perfluorooctanoic acid; Perfluorononic acid; Perfluorobutane sulfonic acid; Perfluoropentanesulfonic acid; Perfluorohexanesulfonic acid; Perfluoroheptane sulfonic acid; Perfluorooctane sulfonic acid; Perfluoronanesulfonic acid; Perfluorodecanesulfonic acid; Perfluoroundecanesulfonic acid; Perfluorododecanesulfonic acid; Perfluorotridecanesulfonic acid; Perfluorooctanesulfonic acid amide; 6:2 Fluorotelomer sulfonic acid; 8:2 Fluorotelomer sulfonic acid; 4:2 Fluorotelomer sulfonic acid; perfluoro-4,8-dioxa-3H-nonanoic acid; Perfluoro-2-propoxypropanoic acid; or 9-chlorohexadecafluoro-3-oxanonane-1-sulfonic acid.
12. A method for regenerating a polymer according to one of claims 1 to 3, a device according to one of claims 4 to 7 or a chromatography column according to claim 8, wherein the polymer is in a state of having PFAS bound, wherein the method comprises the steps of
• rinsing the polymer with an solution thereby regenerating the polymer; wherein the solution is
∘ an aqueous solution with a pH of 8 to 12 or 2 to 6, optionally comprising at least one water soluble salt such as NaCl, ammonium formate or
∘ a C₁ to C₆ alcohol, preferably ethanol, optionally comprising at least one water soluble salt such as NaCl and water, or
∘ a mixture thereof,
• collecting the aqueous solution or solvent containing the PFAS.

13. A method according to claim 12, wherein the aqueous solution or solvent containing the PFAS is concentrated by evaporating the water of solvent, wherein the residue is recycled or pyrolyzed or disintegrated.
